# EUROPÄISCHE PATENTSCHRIFT

(11) **EP 1 450 735 B1**
(45) Veröffentlichungstag und Bekanntmachung des Hinweises auf die Patenterteilung: **02.11.2006**
(21) Anmeldenummer: 01274862.0
(22) Anmeldetag: 05.12.2001
(51) Int. Cl.: A61F 2/44, A61F 2/46

(54) **BANDSCHEIBENPROTHESE ODER NUKLEUS-PROTHESE**
INTERVERTEBRAL DISK PROSTHESIS OR NUCLEUS REPLACEMENT PROSTHESIS
PROTHESE DE DISQUE VERTEBRAL OU PROTHESE DE REMPLACEMENT DE NOYAU

(43) Veröffentlichungstag der Anmeldung: 01.09.2004
(73) Patentinhaber: Synthes GmbH, 4436 Oberdorf (CH)
(72) Erfinder: STUDER, Armin, CH-4513 Langendorf (CH)
(74) Vertreter: Lusuardi, Werther
(86) Internationale Anmeldenummer: PCT/CH2001/000700
(87) Internationale Veröffentlichungsnummer: WO 2003/047472

(56) Entgegenhaltungen:
- EP-A- 1 132 061
- WO-A-00/67650
- WO-A-98/20939
- WO-A-99/02108
- US-A- 5 047 055
- US-A- 5 344 459
- US-A- 5 899 917
- US-A- 6 127 597

## Beschreibung

Die Erfindung betrifft eine Bandscheibenprothese oder Nukleus-Ersatz-Prothese gemäss dem Oberbegriff des Patentanspruchs 1, wie sie beispielsweise aus der EP-A- 11 32 061 bekannt ist.

Aus dem Stand der Technik ist eine grössere Anzahl solcher Bandscheibenprothesen bekannt, welche jedoch alle vorgefertigt sind und im vorgefertigten, relativ voluminösen Zustand in den Zwischenwirbelraum implantiert werden müssen.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung oder ihrer Priorität auch tatsächlich publiziert oder öffentlich bekannt war.

Der Erfindung liegt das Problem zugrunde, eine Bandscheibenprothese oder Nukleus-Ersatz-Prothese zu schaffen, welche in einem dimensionsmässig reduzierten Zustand in den Zwischenwirbelraum implantiert werden kann und dann nach erfolgter Füllung mit einer aushärtbaren, fliessfähigen Masse durch einen Aushärteprozess verfestigt werden kann.

Die Erfindung löst die gestellte Aufgabe mit einer Bandscheibenprothese, welche die Merkmale des Anspruchs 1 aufweist.

Die noch leere Hülle des Bandscheibenimplantates lässt sich leicht im kollabierten Zustand in den Zwischenwirbelraum einführen und dann mittels einer Spritze über eine geeignete Kanüle mit einem fliessfähigen Monomerengemisch füllen. Die Hülle (oder der Ballon) kann an den zur Anlage an die Deckplatten der benachbarten Wirbelkörper bestimmten Seiten eine spezielle Oberfläche und/oder Dicke und/oder ein spezielles Material, z.B. Polycarbonaturethan (PCU) oder Polycarbonat, aufweisen.

Die Vorteile dieser Ausgestaltung bestehen darin, dass die Kontaktflächen zu den beiden Endplatten (Knorpelschicht) der benachbarten Wirbelkörper bei den vorkommenden Bewegungen (Rotation, Extension, Flexion) optimale Bedingungen bezüglich Gleitfähigkeit, Biokompatibilität, Steifigkeit u.s.w. bieten.

Durch eine entsprechende Druckbeaufschlagung kann die Hülle mit dem polymerisierbaren Monomerengemisch soweit aufpumpt werden, dass die Bandscheibenhöhe auf die anatomisch richtige Ursprungshöhe zurückgeführt wird. Dazu kann die Masse mit einem Überdruck von kleiner als 3 Atmosphären, vorzugsweise maximal 1,1 Atmosphären in die Hülle eingebracht werden.
Die Masse kann aber auch im wesentlichen ohne Überdruck in die Hülle eingebracht werden, falls die betroffenen Wirbel mittels geeigneter Instrumente distrahiert werden.

Durch Einführung eines Lichtleiters (z.B. Glasfaserkabel) in die Hülle, bzw. in den Öffnungsbereich der Hülle kann die polymerisierbare Masse beispielsweise mit Blaulicht (z.B. mit 340 nm Wellenlänge) photopolymerisiert werden. Bei wässerigen Monomerlösungen kann durch die polymere Vernetzung ein Hydrogel entstehen.

Damit ist der Vorteil erzielbar, dass das Hydrogel einerseits bei Überbelastung Wasser (Körperflüssigkeit) abgeben kann und anderseits in der Ruhephase Wasser aufnehmen kann. Es resultiert daraus ein Dämpfungseffekt sowie die zusätzliche Möglichkeit die Bandscheibe auf ihre ursprüngliche Höhe zu restaurieren.

Eine bevorzugte Weiterbildung besteht darin, dass die Hülle doppelwandig ausgebildet ist und die aushärtbare, fliessfähige Masse, welche Monomere, Comonomere, Homopolymere, Oligomere oder Gemischen davon enthält, zwischen die beiden Wände eingeführt ist, so dass das Zentrum der Bandscheibenprothese hohl ist. Durch die frei wählbare Grösse des Hohlraumes kann zusätzlich die Flexibilität des Implantates gesteuert werden.

Bei einer weiteren Ausführungsform besteht die Hülle chemisch aus dem gleichen Material wie die in der Hülle sich befindende, aushärtbare, fliessfähige Masse, so dass sich letztere mit dem Hüllenmaterial verbinden kann.

Bei einer weiteren Ausführungsform besteht die Hülle aus einem Material mit Memory-Effekt, so dass sie bei Körpertemperatur eine vorgängig gespeicherte geometrische Form einnimmt.

Bei einer weiteren Ausführungsform enthält die aushärtbare, fliessfähige Masse einen Polymerisationskatalysator und vorzugsweise zusätzlich einen Polymerisationsbeschleuniger.

Bei einer weiteren, bevorzugten Ausführungsform enthält die aushärtbare, fliessfähige Masse einen Photoinitiator, vorzugsweise einen radikalischen Photoinitiator, wobei der Photoinitiator vorzugsweise im Bereich von etwa 340 bis 420 nm Licht absorbiert. Der Photoinitiator kann ein Phosphinoxid, vorzugsweise ein Acylphosphinoxid sein. Das Phosphinoxid kann mit Dimethylacrylamid copolymerisiert sein. Der Vorteil bei der Polymerisation mit Blaulicht gegenüber der Autopolymerisation besteht darin, dass keine erhöhte Wärmeentwicklung entsteht, welche die Eiweissmoleküle zerstören kann. Zudem ist die Handhabung eines Lichtleiters, mit dem das Blaulicht in den Ballon gestrahlt wird, ungefährlich. Die Frequenz und die Dauer der Blaulichtstrahlung kann durch einfache Steuerung an der Lichtquelle eingestellt werden.

Die Monomere, Comonomere, Homopolymere, Oligomere oder Gemische, welche die aushärtbare, fliessfähige Masse enthält, können zweckmässigerweise aus der Gruppe der
a) Polyethylenglykole, vorzugsweise Polyethylenglykol(di)-acrylate;
b) N-vinylpyrrolidone; und
c) Vinyle, vorzugsweise Vinylalkohole; und
d) Styrole
ausgewählt werden.
Die damit erhaltenen Polymerisate können bezüglich ihrer Elastizität in weiten Bereichen variiert werden.

Vorteilhafterweise enthält die aushärtbare, fliessfähige Masse 30 bis 160 Gew.-%, vorzugsweise 40 bis 90 Gew.-% Wasser. Speziell geeignet ist ein Anteil von 45 bis 55 Gew.-% Wasser. Durch die Festlegung wieviel Wasser die polymerisierte Masse, insbesondere wenn letztere ein Hydrogel ist, nachträglich aufnehmen soll (Quellfaktor), kann die zusätzliche Distraktion auf das Wirbelsäulensegment gesteuert werden.

Ein Verfahren zur Herstellung der erfindungsgemässen Bandscheibenprothese oder Nukleus-Ersatz-Prothese umfasst folgende Verfahrensschritte:
A) Implantation einer biokompatiblen Hülle in den Zwischenwirbelraum zweier benachbarter Wirbelkörper;
B) Einführung einer aushärtbaren, fliessfähigen Masse, welche Monomere, Comonomere, Oligomere oder Gemische davon enthält, in das Innere der implantierten biokompatiblen Hülle, wobei die gefüllte Hülle im Zwischenwirbelraum zentriert bleibt; und
C) Aushärtung der in der Hülle befindlichen, aushärtbaren fliessfähigen Masse in situ.

Bei einer Variante des erfindungsgemässen Verfahrens kann die Hülle zwischen Schritt A und B mit Luft aufgeblasen werden. Durch diese Vordistraktion kann eine Überprüfung der Distraktionsfähigkeit des Wirbelsäulensegmentes durchgeführt werden.

Bei einer weiteren Variante des erfindungsgemässen Verfahrens kann die Hülle zwischen Schritt A und B mit einem Röntgenkonstrastmittel gefüllt werden. Das Röntgenkonstrastmittel macht mittels eines Bildwandlers die Position der Hülle im Wirbelsäulensegment sichtbar. Dies ergibt Informationen, ob die Hülle richtig positioniert ist.

Die Aushärtung der Masse kann durch Autopolymerisation oder durch Photopolymerisation erfolgen, vorzugsweise mit sichtbarem oder ultraviolettem Licht.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine zwischen zwei benachbarten Wirbelkörpern implantierte Bandscheibenprothese in der Phase der Auffüllung der Hülle mit einer aushärtbaren, fliessfähigen Masse;
Fig. 2 einen Längsschnitt durch die Bandscheibenprothese nach Fig.1 in der Phase der Aushärtung der fliessfähigen Masse;
Fig. 3 einen Längsschnitt durch eine doppelwandige Bandscheibenprothese;
Fig. 4 einen Längsschnitt durch das Ventil zur Befüllung der Bandscheibenprothese; und
Fig. 5 einen Längsschnitt durch eine Bandscheibenprothese mit unterschiedlich dick ausgebildeten Aussenflächen.

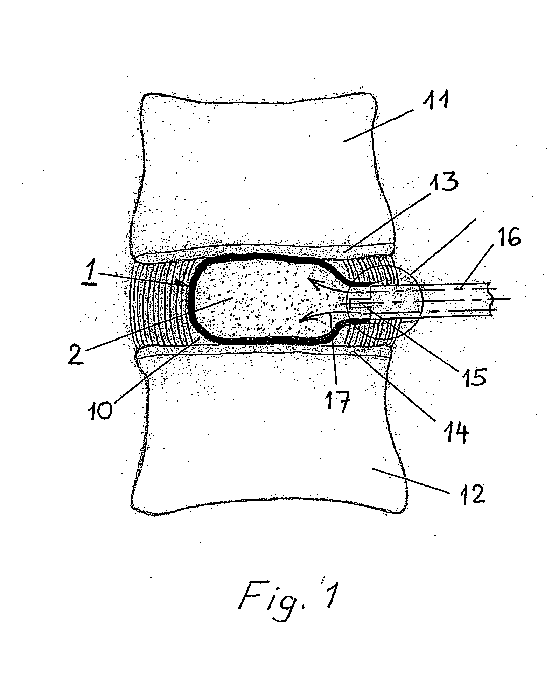

In Fig. 1 ist die Bandscheibenprothese in Form einer Nukleus-Ersatz-Prothese dargestellt und zwar im Zustand, bei welchem die biokompatible Hülle 1 bereits in den Zwischenwirbelraum 10 zweier benachbarter Wirbelkörper 11,12 implantiert ist und über das Ventil 15 und die Kanüle 16 mit einer aushärtbaren, fliessfähigen Masse 2, in Form eines Hydrogels in das Innere der implantierten biokompatiblen Hülle 1 in Richtung der Pfeile 17 aufgefüllt wird. Die gefüllte Hülle 1 bleibt dabei im Zwischenwirbelraum 10 zentriert und legt sich dabei an die beiden Endplatten 13,14 der benachbarten Wirbelkörper 11,12 an.

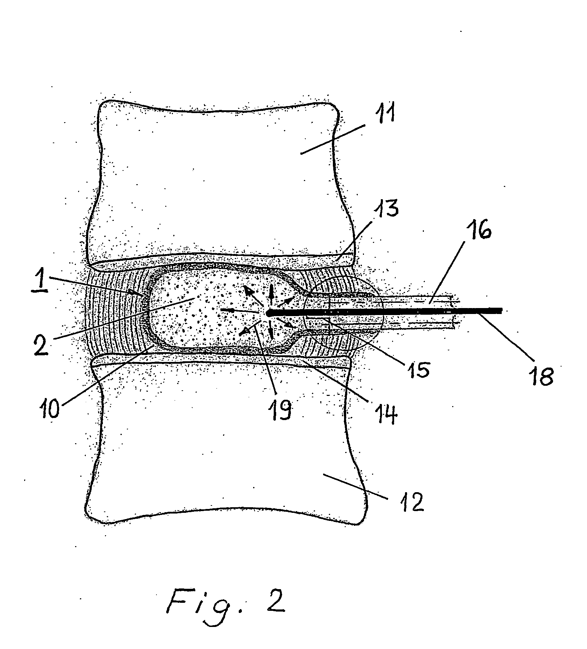

In Fig. 2 ist dargestellt, wie durch Einführung eines Lichtleiters 18 durch die Kanüle 16 in das Innere der implantierten biokompatiblen Hülle 1 die dort befindliche Masse 2 durch Photopolymerisation ausgehärtet wird. Die Masse 2 enthält dazu einen radikalischen Photoinitiator. Das für die Photopolymerisation verwendete Licht ist ultraviolettes Licht, welches durch die Pfeile 19 angedeutet ist. -

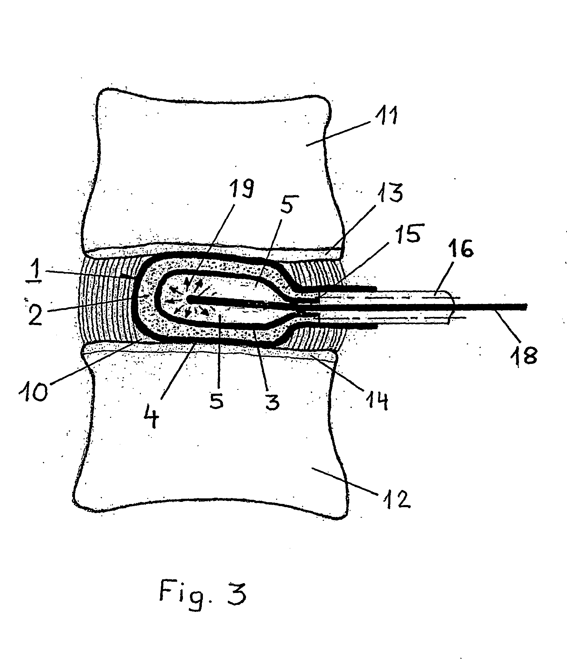

In Fig. 3 ist eine Variante der Bandscheibenprothese dargestellt, bei welcher die Hülle 1 doppelwandig ausgebildet ist und die Masse 2 zwischen die beiden Wände 3,4 eingeführt wird, so dass das Zentrum 5 der Bandscheibenprothese hohl ist.

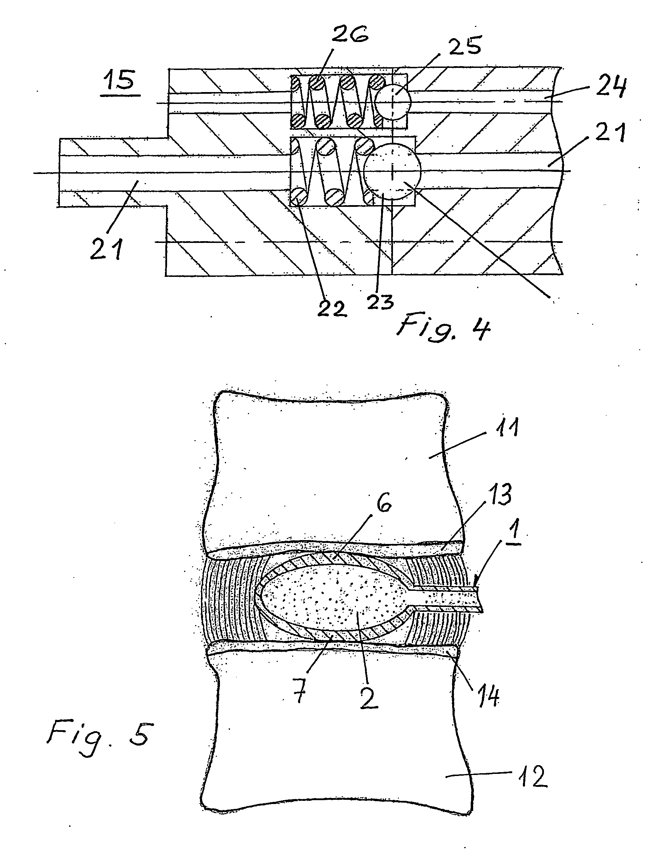

Um sowohl die einwandige als auch die zweiwandige Variante der Bandscheibenprothese mit der Masse 2 befüllen zu können, ist eine spezielles in Figur 4 dargestelltes ventile 15 vorgesehen. Das Ventil 15 besteht im wesentlichen aus einer zentralen Bohrung 21 mit einer von der Feder 22 gehaltenen Kugel 23 als Rückschlagventil und einer peripheren Bohrung 24 mit einer von der Feder 26 gehaltenen Kugel 25 als Rückschlagventil. Die zentrale Bohrung 21 dient zur Befüllung der einwandigen Variante (gemäss den Fig. 1 und 2), die periphere Bohrung 24 dient der Befüllung der zweiwandigen Variante (gemäss Fig. 3). Bei letzterer kann dann die zentrale Bohrung 21 zum Einfüllen von Luft oder von Röntgenkontrastmittel verwendet werden.

In Fig. 5 ist eine weitere Variante der Bandscheibenprothese dargestellt bei welcher die Hülle 1 an den zur Anlage an die Deckplatten 13,14 der benachbarten Wirbelkörper 11,12 bestimmten Wände 6 und 7 dicker ausgebildet sind als in den übrigen Wandbereichen. Mindestens die Wände 6 und 7 der Hülle 1 bestehen aus Polycarbonaturethan (PCU) oder Polycarbonat.

Im folgenden wird die Erfindung anhand einiger Beispiele illustriert.

### Beispiel 1

45 g Polyethylenglykoldiacrylat (PEGDA) mit dem Molekulargewicht 700, sowie 5 g eines Kopolymerisates von 2,6-Dimethyl-3-vinylbenzoylphosphinoxid (DMVBPO) mit Dimethylacrylamid wurden in 50 g destilliertem Wasser gelöst. Die Aushärtung dieses Hydrogels erfolgte mit Blaulicht der Wellenlänge 420 nm mit einer Intensität von 2W/cm².

### Beispiel 2

45 g Polyethylenglykoldiacrylat (PEGDA) mit dem Molekulargewicht 720, sowie 5 g eines Kopolymerisates von 4- (VBPO) mit Dimethylacrylamid wurden in 50 g destilliertem Wasser gelöst. Die Aushärtung dieses Hydrogels erfolgte mit Blaulicht der Wellenlänge 420 nm mit einer Intensität von 2W/cm².

### Beispiel 3

45 g Polyethylenglykoldiacrylat (PEGDA) mit dem Molekulargewicht 750, sowie 5 g eines Kopolymerisates von 2,4,6-Trimethylbenzoylphenyl-4-vinylphenylphosphinoxid (TMBVPO) mit Dimethylacrylamid wurden in 50 g destilliertem Wasser gelöst. Die Aushärtung dieses Hydrogels erfolgte mit Blaulicht der Wellenlänge 420 nm mit einer Intensität von 2W/cm².

## Patentansprüche

1. Bandscheibenprothese oder Nukleus-Ersatz-Prothese mit einer biokompatiblen Hülle (1), in der sich eine aushärtbare, fliessfähige Masse (2) befindet, welche Monomere, Comonomere, Homopolymere, Oligomere oder Gemische davon enthält, **dadurch gekennzeichnet, dass** die Hülle (11) an den zur Anlage an die Deckplatten (13,14) der benachbarten Wirbelkörper (11,12) bestimmten Wänden (6,7) dicker ausgebildet ist als in den übrigen Wandbereichen.

2. Bandscheibenprothese nach Anspruch 1, **dadurch gekennzeichnet, dass** die Masse (2) einen Photoinitiator, vorzugsweise einen radikalischen Photoinitiator enthält.

3. Bandscheibenprothese nach Anspruch 2, **dadurch gekennzeichnet, dass**, der Photoinitiator vorzugsweise im Bereich von etwa 340 bis 420 nm Licht absorbiert.

4. Bandscheibenprothese nach Anspruch 2 oder 3, **dadurch gekennzeichnet, dass** der Photoinitiator ein Phosphinoxid, vorzugsweise ein Acylphosphinoxid ist.

5. Bandscheibenprothese nach Anspruch 4, **dadurch gekennzeichnet, dass** das Phosphinoxid mit Dimethylacrylamid copolymerisiert ist.

6. Bandscheibenprothese nach einem der Ansprüche 1 bis 5, **dadurch gekennzeichnet, dass** die Monomere, Comonomere, Homopolymere, Oligomere oder Gemische davon aus der Gruppe der
a) Polyethylenglykole, vorzugsweise Polyethylenglykol(di)-acrylate;
b) N-vinylpyrrolidone; und
c) Vinyle, vorzugsweise Vinylalkohole; und
d) Styrole
ausgewählt sind.

7. Bandscheibenprothese nach einem der Ansprüche 1 bis 6, **dadurch gekennzeichnet, dass** die Masse (2) 30 bis 160 Gew.-%, vorzugsweise 40 bis 90 Gew.-% Wasser enthält.

8. Bandscheibenprothese nach einem der Ansprüche 1 bis 7, **dadurch gekennzeichnet, dass** die Masse (2) ein Hydrogel enthält.

9. Bandscheibenprothese nach einem der Ansprüche 1 bis 8, **dadurch gekennzeichnet, dass** die Hülle (1) doppelwandig ausgebildet ist und die Masse (2) zwischen die beiden Wände (3,4) eingeführt ist; so dass das Zentrum (5) der Bandscheibenprothese hohl ist.

10. Bandscheibenprothese nach einem der Ansprüche 1 bis 9, **dadurch gekennzeichnet, dass** die Hülle (1) aus einem Material mit Memory-Effekt besteht, so dass sie bei Körpertemperatur eine vorgängig gespeicherte geometrische Form einnimmt.

11. Bandscheibenprothese nach einem der Ansprüche 1 oder 6 bis 10 **dadurch gekennzeichnet, dass** die Masse (2) einen Polymerisationskatalysator und vorzugsweise zusätzlich einen Polymerisationsbeschleuniger enthält.

12. Bandscheibenprothese nach einem der Ansprüche 1 bis 11, **dadurch gekennzeichnet, dass** die Hülle (1) chemisch aus dem gleichen Material besteht, wie die in der Hülle (1) sich befindende Masse (2).

13. Bandscheibenprothese nach einem der Ansprüche 1 bis 11, **dadurch gekennzeichnet, dass** die zur Anlage an die Deckplatten (13, 14) der benachbarten Wirbelkörper (11,12) bestimmten Wände (6,7) aus Polycarbonaturethan (PCU) oder Polycarbonat bestehen.

## Claims

1. An intervertebral disk prosthesis or nucleus replacement prosthesis comprising a bio-compatible pouch (1) receiving a curable, flowable material (2) containing monomers, comonomers, homopolymers, oligomers or mixtures thereof,
**characterized in that**
at the walls (6, 7) designed to rest against the upper plates (13, 14) of the adjacent vertebras (11, 12), the pouch (1) is thicker than in the remaining wall segments.

2. Intervertebral disk prosthesis as claimed in claim 1, **characterized in that** the material (2) contains a photo-initiator, preferably a radicals-generating photo-initiator.

3. Intervertebral disk prosthesis as claimed in claim 2, **characterized in that** the photo-initiator absorbs light preferably in the 340 to 420 nm range.

4. Intervertebral disk prosthesis as claimed in either of claims 2 and 3, **characterized in that** the photo-initiator is a phosphine oxide, preferably an acylphosphine oxide.

5. Intervertebral disk prosthesis as claimed in claim 4, **characterized in that** the phospine oxide is copolymerized with dimethylacryl amide.

6. Intervertebral disk prosthesis as claimed in one of claims 1 through 5, **characterized in that** the monomers, comonomers, homopolymers, oligomers or mixtures thereof are selected from the group of
(a) polyethylene glycols, preferably polyethyleneglycol (di)acrylates;
(b) N-vinylpyrrolidones; and
(c) vinyls, preferably vinyl alcohols; and
(d) styrenes.

7. Intervertebral disk prosthesis as claimed in one of claims 1 through 6, **characterized in that** the material (2) contains by weight 30 to 160 % water, preferably 40 to 90 % water.

8. Intervertebral disk prosthesis as claimed in one of claims 1 through 7, **characterized in that** the material (2) contains a hydrogel.

9. Intervertebral disk prosthesis as claimed in one of claims 1 through 8, **characterized in that** the pouch (1) is double-walled and the material (2) is introduced between the two walls (3, 4) whereby the intervertebral disk prosthesis center (5) is hollow.

10. Intervertebral disk prosthesis as claimed in one of claims 1 through 9, **characterized in that** the pouch (1) is made of a memory-effect substance whereby said pouch shall assume a previously stored geometric shape at body temperature.

11. Intervertebral disk prosthesis as claimed in one of claims 1 or 6 through 10, **characterized in that** the material (2) contains a polymerization catalyst and preferably additionally a polymerization accelerator.

12. Intervertebral disk prosthesis as claimed in one of claims 1 through 11, **characterized in that** the pouch (1) is made of a substance which is chemically identical with the material contained in this pouch.

13. Intervertebral disk prosthesis as claimed in one of claims 1 through 11, **characterized in that**, at its walls (6, 7) touching the upper plates (13, 14) of the adjacent vertebras (11, 12), the pouch (1) is made thicker and preferably consists of polycarbonate urethane (PCU) or polycarbonate.

## Revendications

1. Prothèse de disque intervertébral ou prothèse de remplacement du nucléus, comprenant une enveloppe biocompatible (1) dans laquelle se trouve une masse fluide durcissable (2), laquelle contient des monomères, des comonomères, des homopolymères, des oligomères ou des mélanges de ceux-ci, **caractérisée en ce que** l'enveloppe (1) est plus épaisse au niveau des parois (6, 7) destinées à venir s'appuyer sur les plaques de recouvrement (13, 14) des corps vertébraux vicinaux (11, 12) que dans les autres zones de paroi.

2. Prothèse de disque intervertébral selon la revendication 1, **caractérisée en ce que** la masse (2) contient un photoamorceur, de préférence un photoamorceur radicalaire.

3. Prothèse de disque intervertébral selon la revendication 2, **caractérisée en ce que** le photoamorceur absorbe la lumière de préférence dans la gamme d'environ 340 à 420 nm.

4. Prothèse de disque intervertébral selon la revendication 2 ou 3, **caractérisée en ce que** le photoamorceur est un oxyde de phosphine, de préférence un oxyde d'acylphosphine.

5. Prothèse de disque intervertébral selon la revendication 4, **caractérisée en ce que** l'oxyde de phosphine est copolymérisé avec du diméthylacrylamide.

6. Prothèse de disque intervertébral selon l'une quelconque des revendications 1 à 5, **caractérisée en ce que** les monomères, comonomères, homopolymères, oligomères ou mélanges de ceux-ci sont choisis dans le groupe des
a) polyéthylèneglycols, de préférence des (di)-acrylates de polyéthylèneglycol ;
b) N-vinylpyrrolidones ; et
c) vinyles, de préférence des alcools vinyliques ; et
d) styrènes.

7. Prothèse de disque intervertébral selon l'une quelconque des revendications 1 à 6, **caractérisée en ce que** la masse (2) contient 30% à 160% en poids, de préférence 40% à 90% en poids d'eau.

8. Prothèse de disque intervertébral selon l'une quelconque des revendications 1 à 7, **caractérisée en ce que** la masse (2) contient un hydrogel.

9. Prothèse de disque intervertébral selon l'une quelconque des revendications 1 à 8, **caractérisée en ce que** l'enveloppe (1) est à double paroi et la masse (2) est introduite entre les deux parois (3, 4), de sorte que le centre (5) de la prothèse de disque intervertébral est creux.

10. Prothèse de disque intervertébral selon l'une quelconque des revendications 1 à 9, **caractérisée en ce que** l'enveloppe (1) est composée d'une matière à mémoire de forme, de sorte qu'à la température du corps, elle prend une forme géométrique préalablement mémorisée.

11. Prothèse de disque intervertébral selon l'une quelconque des revendications 1 ou 6 à 10, **caractérisée en ce que** la masse (2) contient un catalyseur de polymérisation et, de préférence, en plus un accélérateur de polymérisation.

12. Prothèse de disque intervertébral selon l'une quelconque des revendications 1 à 11, **caractérisée en ce que** l'enveloppe (1) est chimiquement composée de la même matière que la masse (2) se trouvant dans l'enveloppe (1).

13. Prothèse de disque intervertébral selon l'une quelconque des revendications 1 à 11, **caractérisée en ce que** les parois (6, 7) destinées à venir s'appuyer sur les plaques de recouvrement (13, 14) des corps vertébraux vicinaux (11, 12) sont composées de polycarbonate-uréthanne (PCU) ou de polycarbonate.
